Europäisches Patentamt

European Patent Office

Office européen des brevets

(19)

(11) Publication number: **0 404 533**
**A1**

# EUROPEAN PATENT APPLICATION

(12)

(21) Application number: **90306709.8**

(22) Date of filing: **20.06.90**

(51) Int. Cl.5: **A61K 7/32, A61K 7/34,
A61K 7/38**

(30) Priority: **23.06.89 US 370559**

(43) Date of publication of application:
**27.12.90 Bulletin 90/52**

(84) Designated Contracting States:
**AT BE CH DE DK ES FR GB GR IT LI LU NL SE**

(71) Applicant: **THE PROCTER & GAMBLE
COMPANY**
**One Procter & Gamble Plaza**
**Cincinnati Ohio 45202(US)**

(72) Inventor: **Smith, Scott Edward**
**3992 School Section Road**
**Cincinnati, Ohio 45211(US)**
Inventor: **Ward, Richard Martin**
**7090 Cheyenne Way**
**Mason, Ohio 45040(US)**

(74) Representative: **Brooks, Maxim Courtney et al**
**Procter & Gamble (NTC) Limited Whitley**
**Road Longbenton**
**Newcastle-upon-Tyne NE12 9TS(GB)**

(54) Liquid solubilized antiperspirant actives and processes for preparing the same.

(57) Liquid polyhydric alcohol-solubilized antiperspirant actives comprising at least one antiperspirant active of specified activity selected from aluminum chlorhydroxide salts, zirconyl hydroxychloride salts, zirconium-aluminum-glycine complexes, and mixtures thereof, and at least one polyhydric alcohol, are disclosed. The active selected is dissolved directly into the polyhydric alcohol. The direct mixing of the active in the polyhydric alcohol, without intermediate steps, more effectively maintains the enhanced efficacy status of the active component. Furthermore, without the intermediate steps, it is easier to formulate and manufacture high efficacy antiperspirant/ deodorant compositions which have acceptable cosmetics and reduced visible residue on skin and clothes. The process for preparing these solubilized antiperspirant actives is also disclosed.

EP 0 404 533 A1

# LIQUID SOLUBILIZED ANTIPERSPIRANT ACTIVES AND PROCESSES FOR PREPARING THE SAME

## BACKGROUND OF THE INVENTION

The present invention relates to liquid polyhydric alcohol-solubilized antiperspirant actives. These liquid antiperspirant actives have good antiperspirant activity and cosmetic aesthetics, and are easily processed and formulated into antiperspirant compositions. The present invention also relates to processes for preparing liquid polyhydric alcohol-solubilized antiperspirant actives. This invention further relates to antiperspirant compositions containing the liquid polyhydric alcohol-solubilized antiperspirant actives, and to methods for treating or preventing perspiration and malodor associated with human underarm perspiration using those compositions.

Basic aluminum chlorides are well known in the art as antiperspirant actives. U.S. Patent 2,492,085, Anderson, issued May 6, 1947, and U.S. Patent 2,890,987, Hilfer, issued June 16, 1959, disclose the preparation of such antiperspirant actives. Hilfer teaches that such active salts in an aqueous solution are soluble in polyhydric alcohols.

Since Hilfer, other patents have been issued for processing of aluminum compounds which have superior efficacy. U.S. Patent 3,359,169, Slater, issued December 19, 1967; U.S. Patent 3,420,932, Jones, issued January 7, 1969; U.S. Patent 3,507,896, Jones, issued April 21, 1970; U.S. Patent 3,523,130, Jones, issued August 4, 1970; U.S. Patent 3,555,146, Jones, issued January 12, 1971; all describe methods which use aqueous solutions to create antiperspirant actives. The powdered form of these actives is obtained when the water is evaporated by distillation methods. The use of zirconium compounds instead of aluminum in such processes is described in U.S. Patent 3,555,146, Jones, issued January 12, 1971, and UK Patent Specifications 1,159,685 and 1,159,686, both to Jones, published July 30, 1969.

Many of the forementioned patents teach the addition of amounts of propylene glycol during the processing of actives, in order to make the resulting powder readily soluble in a gellant solubilizer such as propylene glycol when making antiperspirant compositions. U.S. Patent 4,137,306, Rubino, issued January 30, 1979, teaches that powdered aluminum active complexes, not having had propylene glycol added during its processing, must be first dissolved in a monohydric or low boiling point polyhydric alcohol before mixing with the propylene glycol solvent to form a dissolved active antiperspirant stick composition.

The most recent processing methods for making antiperspirant aluminum salts have resulted in significant improvement in their efficacy (so-called "enhanced efficacy antiperspirant actives"). Such methods are described in U.S. Patent 4,359,456, Gosling et al., issued November 16, 1982; UK Patent Application 2,144,992A, published March 20, 1985; European Patent Application 7,191, published January 23, 1980; European Patent Application 274,252, published July 13, 1988, and U.S. Patent 4,775,528, Callaghan, issued October 4, 1988. These references teach that the active salts produced consist of a variety of polymer sizes, with the larger ones being the least efficacious. Therefore, the processes disclosed in these references all seek to minimize the concentration of the larger polymers. These references also disclose an analytical method which identifies the active polymer distribution. The gel permeation chromatography (GPC) analysis, described therein, has identified which polymer distributions are indicative of enhanced efficacy.

Improved methods of water removal have contributed significantly to the enhanced efficacy of the actives described above. U.S. Patent 3,887,692, Gilman, issued June 3, 1975, describes a spray drying method to remove water in the processing of aluminum halide salts. Spray drying the active solution instead of fractionally distilling it (boiling off the water) helps maintain the preferred active polymer distribution obtained through the processing methods disclosed in the references just discussed (in fact, all those references have incorporated spray drying in their active powder processing).

Patents such as Gossling and Callaghan, which teach enhancement of antiperspirant actives, do not disclose any improvements over Rubino with regard to dissolution of actives directly into polyhydric alcohols. This preliminary dissolution of actives in monohydric or low boiling point polyhydric alcohols, as taught in Rubino, results in production of larger polymers and loss of efficacy in antiperspirant compositions made from such.

Therefore, the present invention describes a process where commercially available enhanced aluminum and zirconium antiperspirant actives are disssolved directly into a polyhydric alcohol without pre-dissolving them in monohydric alcohols, low boiling point polyhydric alcohols, or water. The benefits derived include not only simplified processing, but also improved efficacy of the resulting antiperspirant compositions.

In summary, the present invention provides a significant improvement over the art. The goals of the present invention are to provide liquid polyhydric alcohol-solubilized antiperspirant actives which when used in the formulation of antiperspirant compositions produce enhanced efficacy, good antiperspirant activity, good stability with easier manufacturing and formulation, good cosmetics, and little visible residue on skin and clothes. The present invention also provides processes for preparing the liquid polyhydric alcohol-solublized antiperspirant actives without the addition of water, monohydric alcohols or low boiling point polyhydric alcohols. A further object of the present invention is to provide methods for treating or preventing human perspiration and underarm malodor by utilizing compositions containing these solubilized antiperspirant actives.

These and other objects will become readily apparent from the detailed description which follows.

All percentages and ratios used herein are by weight unless otherwise indicated.

## SUMMARY OF THE INVENTION

The present invention relates to liquid polyhydric alcohol-solubilized antiperspirant actives. These liquid antiperspirant actives consist essentially of:

(a) at least one antiperspirant active containing a substantial amount of water associated with it, at a level from about 20% to about 70% of the final solubilized active composition, selected from the group consisting of aluminum chlorhydroxide salts, zirconyl hydroxychloride salts, zirconium-aluminum-glycine complexes, and mixtures thereof, said active having polymer distributions, as determined by gel permeation chromatography, where less than about 15% of the polymers occupy peak 1 and the ratio of peak 4 to peak 3 is greater than about 0.1:1; and

(b) from about 30% to about 80% of at least one polyhydric alcohol.

The present invention further relates to processes for preparing liquid polyhydric alcohol-solubilized antiperspirant actives, wherein the active is not predissolved in water, monohydric alcohols or low boiling point polyhydric alcohols, comprising:

(a) adding with vigorous mixing, at least one powdered antiperspirant active containing a substantial amount of water associated with it, at a level from about 20% to about 70% of the final solubilized active composition, selected from the group consisting of aluminum chlorhydroxide salts, zirconyl hydroxychloride salts, zirconium-aluminum-glycine complexes, and mixtures thereof, said active having polymer distributions, as determined by gel permeation chromatography, where less than about 15% of the polymers occupy peak 1 and the ratio of peak 4 to peak 3 is greater than about 0.1:1 and from about 30% to about 80% of a polyhydric alcohol; and

(b) heating the said solution from about 50° C to about 130° C.

The process may also be practiced by heating the polyhydric alcohol prior to addition of the antiperspirant active.

The present invention also relates to antiperspirant compositions comprising:

(a) from about 1% to about 75% of a liquid polyhydric alcohol-solubilized antiperspirant active of the present invention; and

(b) an antiperspirant carrier.

The present invention finally relates to methods for treating or preventing perspiration and malodor associated with underarm perspiration. These methods comprise applying to the skin of a human a safe and effective amount of a liquid polyhydric alcohol-solubilized antiperspirant active of the present invention.

## DETAILED DESCRIPTION OF THE INVENTION

Liquid Polyhydric Alcohol Solubilized Antiperspirant Actives

The liquid polyhydric alcohol-solubilized antiperspirant actives of the present invention consist essentially of: (a) polyhydric alcohol (preferably glycerine and/or propylene glycol); and (b) an antiperspirant active of specified activity (as defined by peak distribution), containing a substantial amount of water associated with it, selected from aluminum chlorhydroxide, zirconyl hydroxychloride, zirconium-aluminum-glycine complexes, and mixtures thereof.

The specific components of the liquid antiperspirant actives, and their levels, are selected in order to

3

produce a liquid having good viscosity to facilitate formulation of various antiperspirant compositions. These components, and their specific weight percentages and relative ratios, are described in detail immediately hereinafter.

## (a) Polyhydric Alcohols:

The polyhydric alcohols utilized in the present invention comprise aliphatic alcohols which contain from 2 to 12 carbon atoms and 2 or more hydroxyl groups. Examples of such alcohols include propylene glycol, ethylene glycol, diethylene glycol, butylene glycol, higher polyethylene and polypropylene glycols and mixtures thereof. The longer the chain length the less desirable the polyglycols become. The polyhydric alcohols selected are preferably those which are liquid at room temperature. Specific examples of glycols which may be used in the present invention include: 1,2-propylene glycol; 1,3-propylene glycol; 1,3-butylene glycol (1,3-butane-diol); glycerine (1,2,3-trihydroxy propane); 2-methyl-2,4-pentane-diol; 2-ethyl-1,3-hexane-diol, and mixtures thereof. Preferred for use herein are glycerine, propylene glycols, and mixtures thereof. Most preferred is 1,2-propylene glycol.

The polyhydric alcohols of the present invention comprise, in total, from about 30% to about 80%, preferably from about 50% to about 70%, and most preferably from about 50% to about 60%, of the liquid polyhydric alcohol-solubilized antiperspirant actives of the present invention.

## (b) Antiperspirant Actives

Antiperspirant actives useful in the liquid polyhydric alcohol-solubilized antiperspirant actives of the present invention are selected from aluminum chlorhydroxide salts, zirconyl hydroxychloride salts, zirconium-aluminum-glycine complexes, and mixtures thereof with enhanced efficacy due to improved molecular distributions.

Aluminum chlorhydroxide salts useful herein include those of the general formula:

$Al_2(OH)_aCl_b.xH_2O$

wherein a is from about 2 to about 5; $(a + b) = 6$; x is from about 1 to about 6; and wherein a, b, and x may have non-integer values. Particularly preferred are aluminum chlorhydroxides referred to as "5/6 basic chlorhydroxide", wherein $a = 5$; and "2/3 basic chlorhydroxide," wherein $a = 4$. Processes for preparing aluminum salts are disclosed in the following documents, all incorporated by reference herein: U.S. Patent 3,887,692, Gilman, issued June 3, 1975; and U.S. Patent 4,359,456, Gosling et al., issued November 16, 1982.

Zirconyl hydroxychloride salts useful herein include those of the general formula:

$ZrO(OH)_{2-a}Cl_a.xH_2O$

wherein a is from about 1 to about 2, preferably from about 1.5 to about 1.87; x is from about 1 to about 8; and wherein a and x may have non-integer values. These zirconyl hydroxychloride salts are disclosed in Belgium Patent Specification 825,146, Schmitz, issued August 4, 1975, incorporated herein by reference.

Also useful herein are complexes containing zirconium, aluminum and glycine, commonly known as "ZAG complexes". Such ZAG complexes contain aluminum chlorhydroxide and zirconyl hydroxychloride of the formulas detailed above. Preparation of these ZAG complexes is disclosed in the following patent documents, all incorporated by reference herein: U.S. Patent 3,792,068, Luedders et al., issued February 12, 1974; U.S. Patent 4,120,948, Shelton, issued October 17, 1978; and U.S. Patent 4,775,528, Callaghan et al., issued October 4, 1988.

Although the antiperspirant actives are in powder form, they contain an amount of water associated with the metal complex. Such water remains after spray drying, or may be absorbed from the atmosphere after spray drying. It has been discovered that the amount of water associated with the actives described herein has a bearing on the ability of the actives to be solubilized directly into the polyhydric alcohol. A substantial amount of water must be associated with the active in order to permit the direct solubilization of the active into the polyhydric alcohol. For example, when the active is aluminum chlorhydroxide, it is preferred that the water content associated with it be at least about 16% of the active. When the active is zirconyl hydroxychloride, it is preferred that the water content associated with it be at least about 12%, preferably at least about 14%, and most preferably at least about 16%, of the active. When the active is zirconium-aluminum-glycine, the water content associated with it should be at least about 10.5%, preferably at least about 11.5%, and most preferably at least about 12.5%, of the active. Mixtures of such actives may also be used.

The water content of the active is determined by subtracting the percentages of the other elements which make up the active, such as aluminum, chlorine, zirconium and glycine from 100%. The concentrations of the said elements are determined by routine wet chemical analytical methods, such as titration.

In summary, the present invention calls for the dissolution of the improved active powders directly into polyhydric alcohols wherein such mixtures are used in antiperspirant compositions. The actives are not predissolved in water, monohydric alcohols, or low boiling polyhydric alcohols (this is indicated by the use of the phrase "consisting essentially of" in the claims defining the solubilized active). Because the predissolution of the active in a polar solvent has been eliminated, significant loss of efficacy due to the shift in molecular distribution toward the larger polymer species during processing is significantly reduced. In the present invention, the antiperspirant active powders have less than 15% of the active's polymer species occupying peak 1 and a peak 4 to 3 ratio greater than about 0.1:1, before being dissolved in the polyhydric alcohol described herein, as determined by GPC analysis (see Gosling, Callaghan UK Application 2,144,992A, and European Patent Applications 7,191 and 274,252; all cited previously).

The terms "soluble" and "solubilized", as used herein, mean that the antiperspirant active is dissolved in and/or colloidally dispersed (sub-micron particle size) in the polyhydric alcohol of the liquid antiperspirant active composition to give a transparent or semitransparent liquid. Typically, the transparency of the liquid is such that more than about 50%, preferably more than about 75%, of 500 nm light is transmitted through the liquid as measured by a standard UV-visible absorption instrument (relative to liquid polyhydric alcohol without antiperspirant active).

In addition to the water content playing a key role in allowing the direct dissolution into the polyhydric alcohol, it has been found that when used in concentrations under 20%, the enhanced activity antiperspirant actives are not directly soluble in polyhydric alcohols. Such low concentrations of enhanced actives must initially be dissolved in a polar solvent like lower monohydric alcohols or low boiling point polyhydric alcohols to facilitate their dissolution into polyhydric alcohol. It is, therefore, surprising that these actives with the same water content are directly soluble in polyhydric alcohols at higher concentrations, i.e., from about 20% to about 70%, preferably from about 30% to about 50%, and most preferably from about 40% to about 50%, of the total liquid antiperspirant active composition of the present invention.

## Antiperspirant Compositions

In the antiperspirant composition aspect of the present invention, at least one liquid polyhydric alcohol-solubilized antiperspirant active described herein is used in combination with an appropriate carrier. Such compositions may include sticks, sprays, lotions, roll-ons and creams. Antiperspirant sticks are preferred.

Because the actives selected are not preliminarily dissolved in the polar solvents such as low boiling point alcohols or water as described in the art, the enhanced polymer species distribution is maintained when actives are dissolved directly into the propylene glycol solvent. Furthermore, using the solubilized actives of the present invention results in antiperspirant compositions with superior aesthetics to those compositions disclosed in Hilfer, Slater and the Jones patents cited previously.

The liquid polyhydric alcohol-solubilized antiperspirant actives of the present invention typically comprise in total from about 1% to about 75%, preferably from about 10% to about 65%, and most preferably from about 30% to about 55%, of the antiperspirant compositions described herein. The antiperspirant carriers typically comprise in total from about 25% to about 99%, preferably from about 35% to about 90%, and most preferably from about 45% to about 70%, of the antiperspirant compositions of the present invention.

Carriers used in antiperspirant sticks are well known and can be readily selected by one skilled in the art to give desired characteristics such as good aesthetics and high efficacy. Antiperspirant stick carrier components include, but are not limited to, gelling agents, emollients, colorants, perfumes, coupling agents or emulsifiers, monohydric alcohols and fillers. Such components are more fully described in the following publications, the disclosures of which are incorporated by reference herein: Plechner, "Antiperspirants and Deodorants", 2 Cosmetics, Science and Technology 373-416 (Balsam and Sagarin, editors; 1972); Fox, "Gel and Sticks Review and Update", 99 Cosmetics and Toiletries 36-52 (Nov. 1984); Geria, "Formulation of Stick Antiperspirants and Deodorants", 99 Cosmetics and Toiletries, 55-66 (Nov. 1984); and "Gels and Sticks Formulary", 99 Cosmetics and Toiletries, 82-87 (Nov. 1984). The most preferred antiperspirant carriers for use herein are described in U.S. Patent 4,781,917, Luebbe et al., issued November 1, 1988 (incorporated herein by reference). The antiperspirant compositions may also include deodorant actives and antiperspirant actives in addition to the required solubilized actives described above.

## 1. Gelling Agent

The gelling agent selected for use in a stick format plays an essential part in dictating both cosmetic and efficacy advantages. The gelling agent helps to form a firm gel matrix with good consumption characteristics. Typical gelling agents known for use in sticks are selected soaps, waxes and dibenzylaldehyde monosorbitol acetal.

Soaps as gelling agents are described in U.S. Patent 2,857,315, Teller, issued October 21, 1958, and U.S. Patent 2,900,306, Slater, issued August 18, 1954, both of which are incorporated herein by reference. Typically, the sodium and potassium salts of fatty acids containing from about 12 to about 18 carbon atoms are chosen. The most preferred is the sodium salt of stearic acid. These gelling agents generally comprise from about 3% to about 10%, preferably from about 4% to about 8%, of the stick composition. Because soap and aluminum or zirconium actives interact negatively and destabilize the gel matrix, soaps are not preferred for antiperspirant gel sticks.

Wax as a gelling agent is disclosed in the following patent specifications, all of which are incorporated by reference herein: U.S. Patent 4,049,792, Elsnau, issued September 20, 1977; U.S. Patent 4,151,272, Geary et al., issued April 24, 1975; U.S. Patent 4,229,432, Geria, issued October 21, 1980; U.S. Patent 4,280,994, Turney, issued July 28, 1981; U.S. Patent 4,126,679, Davy et al., issued November 21, 1978; and European Patent Application 117,070, May, published August 29, 1984.

Waxy materials are typically incorporated at a level of from about 1% to about 35%, preferably from about 10% to about 30%, of the final composition. Among such waxy materials typically used are the high melting point waxes having a melting point of from about 65°C to 102°C, low melting point waxes having a melting point of from about 37°C to 75°C, and preferably mixtures thereof. High melting point waxes include beeswax, spermaceti, carnauba, bayberry, candelilla, montan, ozokerite, ceresin, paraffin, hydrogenated castor oil (castor wax). synthetic waxes such as Fisher-Tropsch waxes, microcrystalline waxes, and mixtures thereof. Castor wax is a preferred high melting point wax useful herein. Low melting point waxes include fatty acids, fatty alcohols, both fatty acid esters and fatty acid amides having fatty chains of from about 8 to about 30 carbon atoms (preferably from about 12 to about 18 carbon atoms); and mixtures thereof. Preferred low melting point waxes include cetyl alcohol, palmitic acid, cetyl stearate, cetyl palmitate, and mixtures thereof.

Because wax simply suspends the aluminum or zirconium actives as finely divided particles, the composition formed is a chalky white stick, which glides poorly upon application and leaves a visible residue upon the skin. Therefore, wax is not preferred.

In the present invention, dibenzylaldehyde monosorbitol acetal (herein DBMSA) is preferred over soap and wax as the gelling agent for an antiperspirant stick composition. Use of DBMSA gives a stable clear gel when used with metallic antiperspirant actives. DBMSA (commercially available as Millithix 925, manufactured by Milliken Chemical) is described for use as a gelling agent in antiperspirant sticks in U.S. Patent 4,154,816, Roehl et al., issued May 15, 1979; U.S. Patent 4,346,079, Roehl, issued August 24, 1982; and U.S. Patent 4,518,582, Schamper et al., issued May 21, 1985, all of which are incorporated herein by reference. The DBMSA may be used at levels of from about 1.0% to about 15%, preferably from about 1.5% to about 4.5%, of the final composition.

## 2. Solvents

Another component making up the carrier for the antiperspirant stick composition is the solvent for the gelling agents described previously. Typically these solvents are at levels from about 5% to about 85% and selected from the same group of polyhydric alcohols disclosed herein which are used to make the liquid polyhydric alcohol-solubilized antiperspirant actives. Specific examples include but are not limited to propylene glycol, butylene glycol, glycerine and dipropylene glycol. The polyhydric alcohols therefore serve a dual function, solubilizing both the antiperspirant actives as well as the gelling agents which allows the medium to gel into a firm stick.

## 3. Other Components

Stick compositions usually include a variety of ingredients to improve composition, efficacy, stability, cosmetics and aesthetics. Such optional components include but are not limited to emollients, emulsifiers, perfumes, coloring agents and dyes.

In the present invention, emollients are used to provide dry feel and reduce tackiness. These emollients are selected from the group consisting of volatile and non-volatile silicones, fatty alcohols, and $C_{12}$-$C_{15}$ alcohol lactates. The emollient comprises from about 2% to about 30% of the stick composition.

Emulsifiers, or coupling agents, may be selected for use herein, to bring polar and non-polar components of the stick into a homogenous mixture. Emulsifiers include, for example, polyethylene glycol (PEG), polypropylene glycol (PPG), ethers of $C_4$-$C_{22}$ fatty alcohols, and $C_{12}$-$C_{15}$ alcohol lactate. The emulsifier comprises from about 5% to about 60% of the stick composition.

## Methods for Preventing Perspiration and Malodor

The present invention also provides methods for treating or preventing perspiration and malodor associated with human underarm perspiration. These methods comprise applying a safe and effective amount of a liquid polyhydric alcohol-solubilized antiperspirant active of the present invention to the skin in the axillary area of a human. The term "a safe and effective amount", as used herein, is an amount which is effective in eliminating or substantially reducing the production of perspiration which ultimately generates the malodors detected through formation of pungent fatty acids, while being safe for human use at a reasonable risk/benefit ratio. The solubilized active will generally be applied in the form of an antiperspirant composition, such as those described in this application.

## Processes for Preparing Liquid Polyhydric Alcohol-Solubilized Antiperspirant Actives

The present invention further relates to processes for preparing liquid polyhydric alcohol-solubilized antiperspirant actives. These processes comprise combining, with vigorous mixing, at least one powdered antiperspirant active with a substantial amount of water associated with it, at levels from about 20% to about 70% of the final solubilized active composition, selected from the group consisting of aluminum chlorhydroxide salts, zirconyl hydroxychloride salts, zirconium-aluminum-glycine complexes, and mixtures thereof, which have polymer distributions, as determined by gel permeation chromatography, where less than 15% of the polymers occupy peak 1 and the ratio of peak 4 to peak 3 is greater than about 0:1:1, with from about 30% to about 80% of an polyhydric alcohol; and b) heating the said solution from about 50°C to about 130°C. In carrying out this process, the antiperspirant active is not pre-dissolved in water, monohydric alcohols, or low boiling polyhydric alcohols.

The process herein described may also be carried out by heating the polyhydric alcohol to from about 50°C to about 80°C before it is combined, with vigorous mixing, with the powdered antiperspirant active.

In this process, mixing refers to the vigorous combining of separate ingredients by physical means to form a transparent or semi-transparent homogeneous solution such that about 50%, preferably more than about 75%, of 500 nm light is transmitted through the liquid as measured by a standard UV-visible absorption instrument (relative to liquid polyhydric alcohol without antiperspirant active). The method of mixing in this invention includes any means for obtaining the mixture, such as mechanical mixing, homogenization, and shear milling. Most preferable is high turbulent mechanical mixing.

In summary, this process does not include pre-dissolution of the powdered antiperspirant active in a polar solvent prior to its dissolution in the polyhydric alcohol, since such pre-dissolution causes degradation of the preferred molecular distribution of the active. Furthermore, this process allows one to utilize any enhanced active, rather than being restricted to enhanced actives which incorporate a polyhydric alcohol during their manufacture as required in U.S. Patent 4,137,306, Rubino, in order to facilitate dissolution directly into the polyhydric alcohol.

The following examples further describe and demonstrate embodiments within the scope of the present invention. The examples are given solely for the purpose of illustration and are not to be construed as limitations of the present invention as many variations thereof are possible without departing from the spirit or scope.

## EXAMPLE I

## Propylene Glycol Solubilized ACH

Add propylene glycol (200 g; U.S.P. supplied by Union Carbide) to a beaker and heat to 80°C. Slowly add aluminum chlorhydroxide powdered active (200 g Westchlor DM 200 supplied by Westwood Chemical Corporation, having an associated moisture level of at least about 16%) to the beaker while milling with a T-25 Ultra-Turrax (slight warming results from milling). After the addition is complete, the milling is stopped and the solution is cooled (with stirring if desired) to ambient temperature. The resulting transparent liquid contains 50% ACH and 50% propylene glycol.

## EXAMPLE II

### Propylene Glycol Solubilized ACH

Add propylene glycol (200 g; U.S.P. supplied by Union Carbide) to a beaker. Add aluminum chlorhydroxide powdered active (200 g Reach 101, supplied by Reheis Chemical Company, having an associated moisture level of at least about 16%) to the beaker and heat to 80°C. Mill the solution with a T-25 Ultra-Turrax (slight warming results from milling) until transparent at which time the milling is stopped and the solution is cooled (with stirring if desired) to ambient temperature. The resulting transparent liquid contains 50% ACH and 50% propylene glycol.

## EXAMPLE III

### Glycerine Solubilized ACH

Add glycerine (200 g) to a beaker and heat to 80°C. Slowly add aluminum chlorhydroxide powdered active (200 g Reach 101 supplied by Reheis Chemical Company, having an associated moisture level of at least about 16%) to the beaker while milling with a T-25 Ultra-Turrax (slight warming results from milling). After the addition is complete, the milling is stopped and the solution is cooled (with stirring if desired) to ambient temperature. The resulting transparent liquid contains 50% ACH and 50% glycerine.

## EXAMPLE IV

### Propylene Glycol Solubilized ZAG

Add propylene glycol (200 g; U.S.P. supplied by Union Carbide) to a beaker and heat to 80°C. Slowly add aluminum zirconium trichlorohydrex glycine powdered active (200 g Westwood Zr 35B DM supplied by Westwood Chemical Corporation, having an associated moisture level of at least about 10.5%) to the beaker while milling with a Tekmar Tissuemizer. After the addition is complete, the milling is stopped and the solution is cooled (with stirring if desired) to ambient temperature. The resulting transparent liquid contains 50% ZAG and 50% propylene glycol. This composition can be prepared without milling if the ZAG powder is slowly added in small quantities while stirring.

Transparent propylene glycol solubilized ZAG solutions containing various levels of ZAG and propylene glycol are prepared by the same procedure. For a 40% ZAG to 60% propylene glycol solution, use 240 g of propylene glycol and 160 g of the ZAG active. For a 25% ZAG to 75% propylene glycol solution, use 300 g of propylene glycol and 100 g of ZAG active.

## EXAMPLE V

### Propylene Glycol Solubilized ZAG

Add propylene glycol (70,000 g; U.S.P. supplied by Union Carbide) to a vessel and heat to 80°C. Slowly add aluminum zirconium trichlorohydrex glycine powdered active (70,000 g Westwood Zr 35B supplied by Westwood Chemical Corporation, containing an associated moisture level of at least about 10.5%) to the vessel while agitating with an IKA Rototron until all the powder is dissolved. After addition is complete, the agitation is stopped and the solution is cooled (with stirring if desired) to ambient temperature. The resulting transparent liquid contains 50% ZAG and 50% propylene glycol.

## EXAMPLE VI

### Glycerine solubilizedZAG

Add glycerine (300 g) to a beaker and heat to 80°C. Slowly add aluminum zirconium octochlorohydrex glycine powdered active (100 g Wickhen E2357, supplied by Wickhen Products, Inc., containing an associated moisture level of at least about 10.5%) to the beaker while milling with a Tekmar Tissuemizer. After the addition is complete, the milling is stopped and the solution is cooled (with stirring if desired) to ambient temperature. The resulting semi-transparent liquid contains 25% ZAG and 75% glycerine. A 50% ZAG and 50% glycerine solution is made through the same procedure using 200 g of glycerine and 200 g of the ZAG active.

## EXAMPLE VII

An antiperspirant composition of the present invention in stick form, having the components listed below, is prepared as follows:

| Components | Weight % |
|---|---|
| Hexylene Glycol | 20 |
| Propylene Carbonate | 8 |
| $C_{12}$-$C_{15}$ Alcohol Benzoate | 5 |
| Dipropylene Glycol | 34 |
| DBMSA (Millithix) | 3 |
| ACH/Propylene Glycol (50:50 Mixture) made according to Example I | 30 |
| | 100% |

Combine all the ingredients except DBMSA and liquid polyhydric alcohol-solubilized antiperspirant active and heat to about 50°C with mixing until dissolved. Add DBMSA and heat to from about 100°C to about 105°C to form a liquid mixture. Heat the ACH/propylene glycol liquid active of the present invention, made according to Example I, to about 60°C. When the DBMSA mixture has cooled to from about 80°C to about 90°C, add the liquid active and mix for approximately 2 minutes. Pour into stick molds, and let stand for approximately one hour.

This composition, when applied in an effective amount to the axillary area of a human, is effective in treating and preventing perspiration and malodor.

9

**EP 0 404 533 A1**

## Claims

1. Liquid polyhydric alcohol-solubilized antiperspirant actives consisting essentially of:

(a) at least one powdered antiperspirant active containing a substantial amount of water associated with it, at a level from about 20% to about 70% of the final solubilized active composition, selected from the group consisting of aluminum chlorhydroxide salts, zirconyl hydroxychloride salts, zirconium-aluminum-glycine complexes, and mixtures thereof, said active having polymer distributions, as determined by gel permeation chromatography, where less than about 15% of the polymers occupy peak 1, and the ratio of peak 4 to peak 3 is greater than about 0.1:1; and

(b) from about 30% to about 80% of at least one polyhydric alcohol comprising aliphatic alcohols containing from 2 to 12 carbon atoms and 2 or more hydroxyl groups.

2. Liquid polyhydric alcohol-solubilized antiperspirant actives according to Claim 1 wherein the polyhydric alcohol is selected from the group consisting of propylene glycol, glycerine, and mixtures thereof.

3. Liquid polyhydric alcohol-solubilized antiperspirant actives according to Claim 2 consisting essentially of:

(a) from about 30% to about 50% of said antiperspirant active; and

(b) from about 50% to about 70% of said polyhydric alcohol.

4. Liquid polyhydric alcohol-solubilized antiperspirant actives according to Claim 3 consisting essentially of:

(a) from about 40% to about 50% of said antiperspirant active; and

(b) from about 50% to about 60% of said polyhydric alcohol.

5. Liquid polyhydric alcohol-solubilized antiperspirant actives according to Claim 1 wherein the actives are selected from the group consisting of:

(a) aluminum chlorhydroxide salts having at least about 16% water associated therewith;

(b) zirconyl hydroxychloride salts having at least about 12% of water associated therewith;

(c) zirconium-aluminum-glycine salts having at least about 10.5% water associated therewith; and

(d) mixtures thereof.

6. Liquid polyhydric alcohol-solubilized antiperspirant actives according to Claim 5 wherein the polyhydric alcohol is 1,2-propylene glycol.

7. Antiperspirant compositions comprising:

(a) from about 1% to about 75% of a liquid polyhydric alcohol-solubilized antiperspirant active according to Claim 1; and

(b) an antiperspirant carrier.

8. Antiperspirant compositions comprising:

(a) from about 1% to about 75% of a liquid polyhydric alcohol-solubilized antiperspirant active according to Claim 3; and

(b) an antiperspirant carrier.

9. Antiperspirant compositions comprising:

(a) from about 1% to about 75% of a liquid polyhydric alcohol-solubilized antiperspirant active according to Claim 5; and

(b) an antiperspirant carrier.

10. An antiperspirant stick composition comprising:

(a) from about 1% to about 75% of a liquid polyhydric alcohol-solubilized antiperspirant active according to Claim 5;

(b) from about 1% to about 15% of a gelling agent;

(c) from about 5% to about 85% of a gellant solubilizer;

(d) from about 2% to about 30% of an emollient; and

(e) from about 5% to about 60% of an emulsifier.

11. A method for treating or preventing perspiration and malodor associated with human underarm perspiration, comprising applying to the skin of a human a safe and effective amount of a liquid polyhydric alcohol-solubilized antiperspirant active according to Claim 1.

12. A process for preparing liquid polyhydric alcohol-solubilized antiperspirant actives, wherein the active is not pre-dissolved in water, monohydric alcohols, or low boiling point polyhydric alcohols, comprising:

(a) combining with vigorous mixing at least one powdered antiperspirant active containing a substantial amount of water associated with it, at a level from about 20% to about 70% of the final solubilized active composition, selected from the group consisting of aluminum chlorhydroxide salts, zirconyl hydroxychloride salts, zirconium-aluminum-glycine complexes, and mixtures thereof, said active having polymer distribu-

10

EP 0 404 533 A1

tions, as determined by gel permeation chromatography, where less than about 15% of the polymers occupy peak 1, and the ratio of peak 4 to peak 3 is greater than about 0.1:1, with from about 30% to about 80% of at least one polyhydric alcohol comprising aliphatic alcohols containing from 2 to 12 carbon atoms and 2 or more hydroxyl groups; and

(b) heating said solution from about 50°C to about 130°C.

13. A process for preparing liquid polyhydric alcohol-solubilized actives according to Claim 12 wherein the polyhydric alcohol is selected from the group consisting of propylene glycol, glycerine, and mixtures thereof.

14. A process for preparing liquid polyhydric alcohol solubilized actives according to Claim 13 wherein the polyhydric alcohol is 1,2 propylene glycol.

15. A process for preparing liquid polyhydric alcohol-solubilized antiperspirant actives, wherein the active is not pre-dissolved in water, monohydric alcohols, or low boiling point polyhydric alcohols, comprising:

(a) heating at least one polyhydric alcohol comprising aliphatic alcohols containing from 2 to 12 carbon atoms and 2 or more hydroxyl groups, at a level from about 30% to about 80% of the final solubilized active composition, to from about 50°C to about 130°C; and

(b) vigorously mixing at least one powdered antiperspirant active containing a substantial amount of water associated with it, at a level from about 20% to about 70% of the final solubilized active composition, selected from the group consisting of aluminum chlorhydroxide salts, zirconyl hydroxychloride salts, zirconium-aluminum-glycine complexes, and mixtures thereof, said active having polymer distributions, as determined by gel permeation chromatography, where less than about 15% of the polymers occupy peak 1, and the ratio of peak 4 to peak 3 is greater than about 0.1:1, with said polyhydric alcohol.

16. A process for preparing liquid polyhydric alcohol-solubilized actives according to Claim 15 wherein the polyhydric alcohol is selected from the group consisting of propylene glycol, glycerine, and mixtures thereof.

17. A process for preparing liquid polyhydric alcohol-solubilized actives according to Claim 16 wherein the polyhydric alcohol is 1,2 propylene glycol.

18. A process for preparing liquid polyhydric alcohol-solubilized actives according to Claim 16 wherein the mixture contains from about 40% to about 50% of said antiperspirant actives selected from the group consisting of:

(a) aluminum chlorhydroxide salts having at least about 16% water associated therewith;

(b) zirconyl hydroxychloride salts having at least about 12% of water associated therewith;

(c) zirconium-aluminum-glycine salts having at least about 10.5% water associated therewith; and

(d) mixtures thereof.

19. A liquid polyhydric alcohol-solubilized antiperspirant active prepared according to the process of Claim 12.

20. A liquid polyhydric alcohol-solubilized antiperspirant active prepared according to the process of Claim 15.

| | DOCUMENTS CONSIDERED TO BE RELEVANT | | EP 90306709.8 |
|---|---|---|---|

| Category | Citation of document with indication, where appropriate, of relevant passages | Relevant to claim | CLASSIFICATION OF THE APPLICATION (Int Cl⁵) |
|---|---|---|---|
| X | EP - A2 - 0 295 071 <br> (THE PROCTER & GAMBLE CO.) <br> * Pages 3,4; examples; claims * | 1-11, 15-18, 20 | A 61 K 7/32 <br> A 61 K 7/34 <br> A 61 K 7/38 |
| X | EP - A2 - 0 295 070 <br> (THE PROCTER & GAMBLE CO.) <br> * Claims; page 3, line 30 - page 4, line 21; page 5, lines 31-50; examples * | 1-20 | |
| A | DE - A - 1 617 491 <br> (H. LEIBIGER) <br> * Page 3, lines 14-28; page 4, line 30 - page 5, line 9 * | 1-5,7, 8,9, 11,15, 18,20 | |
| D,A | US - A - 2 890 987 <br> (H. HILFER) <br> * Claims; column 1, line 64 - column 2, line 59 * | 1-20 | |

TECHNICAL FIELDS
SEARCHED (Int Cl⁵)

A 61 K 7/00

The present search report has been drawn up for all claims

| Place of search | Date of completion of the search | Examiner |
|---|---|---|
| VIENNA | 05-10-1990 | IRMLER |

CATEGORY OF CITED DOCUMENTS

X  particularly relevant if taken alone
Y  particularly relevant if combined with another document of the same category
A  technological background
O  non-written disclosure
P  intermediate document

T  theory or principle underlying the invention
E  earlier patent document but published on, or after the filing date
D  document cited in the application
L  document cited for other reasons

&  member of the same patent family, corresponding document

EPO Form 1503 03 82